# EUROPEAN PATENT APPLICATION

(11) **EP 4 557 296 A1**
(43) Date of publication of application: **21.05.2025**
(21) Application number: 24213281.9
(22) Date of filing: 15.11.2024
(51) Int. Cl.: G16B 20/00, G16B 40/20, G16H 20/10, G16H 50/20

(54) **METHODS AND SYSTEMS FOR DETERMINING HER2 STATUS USING MOLECULAR DATA**

(30) Priority: 15.11.2023 US 202363599508 P
(71) Applicant: Tempus AI, Inc., Chicago, IL 60654 (US)
(72) Inventor: Ahmed, Talal, Highland Park, 08904 (US); Pelossof, Raphael, New York, 10044 (US); Carty, Mark, Brooklyn, 11203 (US); Nadhamuni, Kaveri, Brooklyn, 11226 (US)
(74) Representative: Hofmann, Matthias

(57) **Abstract**

A computer-implemented method, computing system and computer-readable medium for determining HER2-low status of a patient using molecular data of the patient includes: (a) receiving digital biological data; (b) processing the digital biological data using a trained multistage machine learning architecture; (c) generating a digital HER2-low status report corresponding to the patient; and (d) causing the digital HER2-low status report to be displayed. A computer-implemented method, computing system and computer-readable medium for training a model architecture to determine HER2-low status of a patient using molecular data of the patient includes: (a) receiving training digital biological data; (b) initializing a machine learning model; (c) processing the plurality of molecular signatures using the machine learning model to generate a trained machine learning model; and (d) storing the trained machine learning.

## Description

### CROSS-REFERENCE TO RELATED APPLICATIONS

The present application claims priority to U.S. Patent Application No. 63/599,508, entitled METHODS AND SYSTEMS FOR DETERMINING HER2 STATUS USING MOLECULAR DATA, filed on November 15, 2023, and hereby incorporated by reference in its entirety.

### FIELD

The present disclosure is directed to methods and systems for determining HER2 status using molecular data, and more particularly, to techniques for training and operating one or more machine learning models to process molecular data of a patient to predict HER2 status, including HER2-low status.

### BACKGROUND

The background description provided herein is for the purpose of generally presenting the context of the disclosure. Work of the presently named inventors, to the extent it is described in this background section, as well as aspects of the description that may not otherwise qualify as prior art at the time of filing, are neither expressly nor impliedly admitted as prior art against the present disclosure.

Human epidermal growth factor receptor 2 (HER2), is a protein that can play a significant role in certain types of cancer (e.g., breast cancer). HER2 is a receptor protein found on the surface of some normal cells that can be overexpressed in cancer cells. When HER2 is overexpressed, it can lead to uncontrolled cell growth and the development of aggressive tumors. A patient's HER2 status is an important factor in the diagnosis and treatment of cancer. To determine HER2 status, tumors are conventionally classified into one of the following groups:
1. HER2-Negative (HER2-): This group includes cancer tumors that do not overexpress the HER2 protein or have very low levels of HER2 expression. HER2-negative cancer is usually not treated with drugs specifically targeting HER2.
2. HER2-Positive (HER2+): These tumors overexpress the HER2 protein, and this overexpression contributes to the rapid growth and aggressiveness of the cancer. HER2-positive cancer is more likely to respond to targeted therapies that block the HER2 receptor, such as trastuzumab (Herceptin) and other HER2-targeted drugs. These targeted therapies can be used in addition to standard treatments like chemotherapy, surgery, and radiation therapy.
3. HER2-Equivocal: In some cases, the level of HER2 expression in a tumor may be borderline or equivocal, meaning it's neither clearly positive nor negative. In such cases, further testing, such as fluorescence in situ hybridization (FISH), chromogenic in situ hybridization (CISH), and/or immunohistochemistry (IHC) may be conducted to determine the HER2 status more definitively. In general, FISH and IHC are the standard methods that are conventionally used to determine HER2 status.

More recently, a new classification called HER2 low (sometimes referred to as HER2-low or HER2-2+) was developed to characterize the patient's HER2 status. This category is distinct from HER2-negative and HER2-positive cancers and is used to describe tumors with lower levels of HER2 expression. HER2-low cancer is characterized by having HER2 expression that falls into an intermediate range, meaning it is higher than HER2-negative (HER2-) but not high enough to be classified as HER2-positive (HER2+). HER2-low tumors typically have HER2 protein expression that is low to moderate and are generally not amplified. The classification of HER2-low breast cancer has gained importance because it has been identified as a distinct subgroup with its own characteristics and potential treatment implications. Because the importance of the HER2-low category was not widely recognized previously, traditional HER2 tests were not designed to accurately classify patients into the HER2-low category. While HER2-low tumors are not as responsive to traditional HER2-targeted therapies like trastuzumab (Herceptin) that are effective in HER2-positive breast cancer, recent research has shown that some experimental targeted therapies may be beneficial for HER2-low breast cancer.

One such experimental therapy is trastuzumab deruxtecan (T-DXd), which has shown promise in clinical trials for treating HER2-low breast cancer. This drug is an antibody-drug conjugate that specifically targets HER2 and delivers a chemotherapy drug to cancer cells, making it a potential treatment option for HER2-low breast cancer.

However, there is currently no effective means of identifying patients who have HER2 protein expression that will benefit from that drug. At first glance, IHC and FISH seem like they might be candidates for detecting HER2-low status, but IHC and FISH suffer from several technical drawbacks. First, IHC and FISH require multiple tissue slides (one and three or more, respectively). In many cases, there is not sufficient slide material (e.g., tumor block) available to perform IHC and/or FISH in addition to (or instead of) any other uses of the tissue. Further, analysis using IHC and FISH is on a single protein basis, and a single slide must generally be used for each protein. Inputs to IHC and FISH are generally fixed-formalin paraffin-embedded (FFPE) stained tumor block slides. Thus, the number of microscope slides generally determines the number of tests that may be run. For example, running IHC requires one slide, FISH requires more slides.

Further, expensive antibodies are required for IHC. Staining takes time, and pathology review is generally manually performed and takes significant time for assessment.

Still further, the precision and consistency of IHC/FISH interpretation is lacking. For example, one pathologist may annotate a slide as HER2-2+, while another annotates a slide as HER2-1+. Herein, in general, IHC 0 is her2-neg, IHC 1+ is her2-low, IHC 2+ and FISH neg is her2-low, IHC 2+ and FISH pos is her2-positive, IHC 3+ is her2-positive. In such cases, the results are not useful for downstream processes. Overall, the determination of HER2-low status via IHC has been reported to have high variability, given the high level of discordance among pathologists on HER2 IHC status, especially for determination of HER2-low status (and in particular, the differentiation of HER2-low and HER2-negative statuses, respectively).

Inconsistent annotation can negatively affect clinical practice in the fields of pathology, oncology, and disease diagnostics, by leading to discrepancies in interpretation and diagnosis, potentially affecting the diagnosis and classification of disease. For example, in cancer diagnosis, inconsistent annotation of tumor markers can result in misclassification, leading to incorrect treatment decisions. Inconsistent annotation also has the potential to lead to under-treatment or over-treatment, potentially negatively affecting patient outcomes and quality of care. Inconsistent annotation in research can compromise the reproducibility of scientific findings, preventing replication or validation of research results. Inconsistent annotation may have negative effects in the areas of data quality, biomarker discovery, quality control and resource efficiency.

Inconsistent annotation can also be caused by simple confusion of numeric scoring in the different ways that HER2 status is labeled. In the past, pathologists were trained to equate 0 and 1 IHC scores with HER2-Negative. The number 2 represented an HER2-Equivocal, and then if subsequent FISH testing was positive, these results were labeled HER2-Positive; and if the subsequent FISH testing was negative, these results were labeled as HER2-Negative. The number 3 was categorized as HER2-Positive. In view of the fourth category (HER2-low), 0 is now interpreted as HER2-Negative, 1 is interpreted as HER2-low, and 2 and 3 are still categorized the same way (IHC 2+, FISH negative is HER2-low IHC 2+, FISH positive is HER2-positive). Having two systems in which numeric scores can refer to different meanings has caused confusion among the pathologist community.

Moreover, IHC and FISH tests are not routinely used to assess HER2 status for all cancers, so these values are not always available for analysis. For example, with some gastrointestinal cancers, IHC and FISH are typically not part of the clinical workflow. Thus, data may not be available for modeling. Metastatic breast cancer that spreads to the gastrointestinal tract may not be tested for HER2 because the clinician does not know that this metastatic cancer is breast cancer. Many patients may have an RNA/DNA sample but no IHC result. And still further, FISH is much more expensive than IHC, due to more slides being needed to execute FISH.

More accurate determination of which patients are likely to respond to HER2 targeted therapies is important because it helps guide treatment decisions for cancer patients. For example, HER2-targeted therapies have been shown to be highly effective in HER2-positive breast cancer, as determined by IHC/FISH, improving both survival rates and treatment outcomes. In contrast, HER2-negative breast cancer patients are typically treated with other therapies, and HER2-targeted drugs are not used because they are unlikely to be beneficial. However, recent studies have shown that HER2-low patients, who were traditionally identified as subset of the HER2-negative population, may also benefit from HER2-targeted therapies and there is a need for more accurate methods for differentiating HER2-low patients from the patient population with no HER2 protein expression.

Accordingly, there is an opportunity for improved platforms and technologies for determining HER2 status using molecular data, by enhancing the reproducibility, precision, and sensitivity of HER2 status testing.

### SUMMARY

In an aspect, a computer-implemented method for determining HER2-low status of a patient using molecular data of the patient includes: (a) receiving, via one or more processors, digital biological data; (b) processing, via one or more processors, the digital biological data corresponding to the patient using a trained multi-stage machine learning architecture, wherein the processing includes: (i) processing the digital biological data using a trained HER2-positive model to determine whether the digital biological data indicates that a HER2 status of the patient is HER2-positive; (ii) when the HER2 status of the patient is not HER2-positive, processing the digital biological data using a trained HER2-low model to identify whether the HER2 status of the patient is HER2-low; and (iii) when the HER2 status of the patient is not HER2-positive or HER2-low, designating the HER2 status of the patient as HER2-negative; (c) generating, via one or more processors, a digital HER2-low status report corresponding to the patient; and (d) causing, via a display device, the digital HER2-low status report to be displayed.

In another aspect, a computer-implemented method for training a model architecture to determine HER2-low status of a patient using molecular data of the patient includes (a) receiving, via one or more processors, training digital biological data, the training digital biological data including a plurality of molecular signatures, each having a respective label; (b) initializing, via one or more processors, a machine learning model in a memory of a computer, the machine learning model having a plurality of hyperparameters; (c) processing, via one or more processors, the plurality of molecular signatures in the training digital biological data and the respective label of each of the plurality of molecular signatures using the machine learning model to generate a trained machine learning model; and (d) storing, via one or more processors, the trained machine learning model in a memory of a computer, wherein the storing includes generating a serialized copy of the machine learning model, and writing the serialized copy of the machine learning model to the memory of the computer.

In yet another aspect, a computing system includes one or more processors; and one or more memories having stored thereon computer-readable instructions that, when executed, cause the computing system to: (a) receive digital biological data; (b) process the digital biological data corresponding to a patient using a trained multi-stage machine learning architecture, wherein the processing includes: (i) processing the digital data using a trained HER2-positive model to determine whether the digital biological data indicates that a HER2 status of the patient is HER2-positive; (ii) when the patient is not HER2-positive, processing the digital biological data using a trained HER2-low model to identify whether the HER2 status of the patient is HER2-low; and (iii) when the patient is not HER2-positive or HER2-low, designating the HER2 status of the patient as HER2-negative; (c) generate, via one or more processors, a digital HER2-low status report corresponding to the patient; and (d) causing, via a display device, the digital HER2-low status report to be displayed.

In still another aspect, a computer-readable medium includes computer-executable instructions that, when executed, cause a computer to: (a) receive digital biological data; (b) process the digital biological data corresponding to a patient using a trained multi-stage machine learning architecture, wherein the processing includes: (i) processing the digital data using a trained HER2-positive model to determine whether the digital biological data indicates that a HER2 status of the patient is HER2-positive; (ii) when the patient is not HER2-positive, processing the digital biological data using a trained HER2-low model to identify whether a HER2 status of the patient is HER2-low; and (iii) when the patient is not HER2-positive or HER2-low, designating the HER2 status of the patient as HER2-negative; (c) generate, via one or more processors, a digital HER2-low status report corresponding to the patient; and (d) cause, via a display device, the digital HER2-low status report to be displayed.

In a further aspect, a computing system includes one or more processors; and one or more memories having stored thereon computer-readable instructions that, when executed, cause the computing system to: (a) receive training digital biological data, the training digital biological data including a plurality of molecular signatures, each having a respective label; (b) initialize a machine learning model in a memory of a computer, the machine learning model having a plurality of hyperparameters; (c) process the plurality of molecular signatures in the training digital biological data and the respective label of each of the plurality of molecular signatures using the machine learning model to generate a trained machine learning model; and (d) store the trained machine learning model in a memory of a computer, wherein the storing includes generating a serialized copy of the machine learning model, and writing the serialized copy of the machine learning model to the memory of the computer.

In yet another aspect, a computer-readable medium includes computer-executable instructions that, when executed, cause a computer to: (a) receive training digital biological data, the training digital biological data including a plurality of molecular signatures, each having a respective label; (b) initialize a machine learning model in a memory of a computer, the machine learning model having a plurality of hyperparameters; (c) process the plurality of molecular signatures in the training digital biological data and the respective label of each of the plurality of molecular signatures using the machine learning model to generate a trained machine learning model; and (d) store the trained machine learning model in a memory of a computer, wherein the storing includes generating a serialized copy of the machine learning model, and writing the serialized copy of the machine learning model to the memory of the computer.

### BRIEF DESCRIPTION OF THE DRAWINGS

The figures described below depict various aspects of the system and methods disclosed herein. It should be understood that each figure depicts an example of aspects of the present systems and methods.
FIG. 1 depicts an exemplary computing system for determining HER2 status using molecular data, according to some aspects.
FIG. 2 depicts an exemplary multi-stage machine learning model architecture, according to some aspects.
FIG. 3A depicts an exemplary HER2 status digital report, according to some aspects.
FIG. 3B depicts an exemplary progesterone receptor positive (PR+) status and estrogen receptor (ER) status digital report, according to some aspects.
FIG. 3C depicts an exemplary digital report of a patient's gene rearrangement and altered splicing analysis from RNA sequencing, according to some aspects.
FIG. 4 depicts an exemplary flow diagram of a computer-implemented method for determining HER2-low status of a patient using molecular data of the patient, according to some aspects.
FIG. 5 depicts an exemplary flow diagram of a computer-implemented method for training a model architecture to determine HER2-low status of a patient using molecular data of the patient, according to some aspects.

### DETAILED DESCRIPTION

### Overview

The present techniques are directed to methods and systems for determining HER2 status using molecular data of a patient, and more particularly, to methods and systems for training and operating one or more models to process molecular data of a patient to predict HER2 status (as defined by IHC/FISH testing). The present techniques enable a clinician to receive a predictive score that a given patient would have a HER2-low status if evaluated using IHC and FISH. This advantageously enables the clinician to have a comparable result to IHC and FISH testing, without actually performing those tests, which saves on material costs and avoids potential inaccuracies of those tests, or alerts the clinician that a patient who would not normally receive HER2 testing may benefit from HER2 testing, as described above.

In some aspects, the present techniques may generate a molecular signature using one or more portions of the patient molecular data. For example, copy number variants (CNV) data and/or RNA data may be joined, via concatenation or otherwise to form the molecular signature. The present techniques may generate the molecular signature using additional/different data, in some aspects. For example, features included in the molecular signature may include DNA data in some aspects.

As noted, IHC and FISH may lead to inconsistently annotated slides, and may not be usable in cases where there is insufficient tumor block. Thus, an advantage of using molecular tests as in the present techniques is that if one has already performed RNASeq, a predictor may be run on RNASeq data and/or DNASeq data to predict HER2 status, regardless of whether additional tissue is available-as noted above, not only may tissue be in short supply, IHC and FISH tests are not routinely used to assess HER2 status for all cancers, so these values are not always available for analysis. Many patients may have an RNA/DNA sample but no IHC result, and the present techniques advantageously expand the patient population that may be eligible for HER2-based targeted therapies. The present techniques thus have enhanced capabilities over conventional techniques. Another advantage of the present molecular testing is that the problem of inconsistent annotation is solved. Still further, the present techniques may process all 20,000 genes at a time using RNASeq, which avoids the bottleneck of IHC and FISH, in which each run of the test is generally limited to fewer than 50 genes.

As discussed, a FISH test is much more expensive than an IHC test (more cost, more slides needed to execute FISH). Thus, the present techniques are particularly compelling for the IHC 2+ cases. In future aspects, when modeling more proteins beyond HER2, the present techniques will not require additional tumor slides to re-execute the IHC test for different proteins (e.g., TROP2).

The present techniques may include a feedback mechanism, whereby drug response data of one or more patients whose molecular data is tested to determine an HER2 status prediction is fed back into a model training pipeline, enabling the model to learn better criteria for predicting HER2 status of a patient. Having molecular modeling with feedback may help to fine-tune thresholds, by reconciling ground truth from future results with past predictions. Specifically, it is envisioned that RNA-seq training data may be enriched with data indicating whether the patient responded to HER2-targeted therapy (was patient a responder or not), which may be used as a training target instead of IHC/FISH-determined HER2 status.

In some aspects, the present techniques may use slides as do IHC and FISH to obtain sequencing data (e.g., by scraping). In some aspects, use of slides may be entirely skipped or omitted. For example, biological patient data may be taken directly from surgical reception or a biopsy, and nucleic acids isolated from that material directly. The present techniques do not require staining. Tissues are degraded by liquid buffers and enzymes to enable RNA and/or DNA to be isolated and prepared for sequencing machines, which generate RNASeq and/or DNAseq data. CNVs may be determined from DNA data and/or inferred from RNA data (for example, amplification/gain CNVs may be associated with high RNA expression levels and loss CNVs may be associated with low RNA expression levels. Generally, CNV data represents missing or extra copies of chromosomes in cells, as seen in cancer cells. RNASeq and CNV data may be used to predict HER2 status, because HER2 positivity can be related to copy number gain in the HER2 gene. Thus, the present techniques are not as destructive as conventional techniques, because slides are not being consumed or used as would be the case during conventional IHC or FISH tests. The present techniques are also advantageous because they do not require the expense of antibodies, stains, testing slides and other materials. Further, the present techniques do not require a human to be "in the loop" of the predictive algorithm, as IHC and FISH often do. Moreover, the present techniques are more deterministic, insofar as that the same RNAseq and DNAseq data (or derivatives like CNV data) processed by the present modeling will give the same answer if run sequentially (or different answers that are correctable) whereas human pathologist analysis may not, as discussed above. A tumor block does not always give the exact same RNAseq results (because of variability/fluctuations in the machine). Usually these fluctuations do not affect modeling outputs, unless the sample is close to the threshold. However, a particular set of RNAseq/DNAseq data will always get the same output from the present modeling techniques.

In particular, because HER2 status of 0 and 1 were both categorized as HER2-Negative in the past, sometimes pathologists were less careful about distinguishing between 0 and 1 IHC scores. Another benefit of the present techniques is that the RNASeq analysis is not affected by human subjectivity, and even if a patient in the training data has been incorrectly scored as a 0 and actually should be a 1+ (or *vice versa*)*,* the present machine learning-based techniques will be able to accurately categorize the patient.

In some examples, CNV data alone can distinguish HER2 positives from not-HER2 positives well, but has significant dynamic range. The present multi-modal techniques that use both CNV and RNASeq data to predict HER2 status, enable more accurate prediction of HER2 positives and HER2 low subtypes.

Further, in some aspects, data of patients previously assigned a HER2-negative status could be processed using the stage two portion (second stage) of the model architecture, to identify those patients that are HER2-low. Thus, in some aspects, only the second portion of the architecture may be used, if another technique has already been used to identify the patient's HER2-positive/ HER2-negative status, or if HER2-positives are not of interest for other reasons. In this example, any HER2-positives included in the data provided to the stage two model may be classified as HER2-low. In this case, HER2-low would not be an accurate label, but another label could be used (e.g., responder/ non-responder- referring to a patient whose sample is either HER2-positive or HER2-low).

In some examples, there is not a perfect correlation between IHC/FISH status and drug response. In some aspects, instead of using IHC/FISH for training, drug response data may be used for training. After drug response data becomes available, considering discordance between IHC/FISH labels and molecular subtypes and response data may provide more clues. Questions that may be answered include 1) whether there are IHC/FISH subtypes that better explain or predict drug response; 2) whether there are molecular subtypes that explain or predict drug response better; and/or 3) whether there are a cascade of IHC/FISH labels and molecular subtypes that explain or predict drug response better.

### Exemplary Computer-Implemented Machine Learning Models

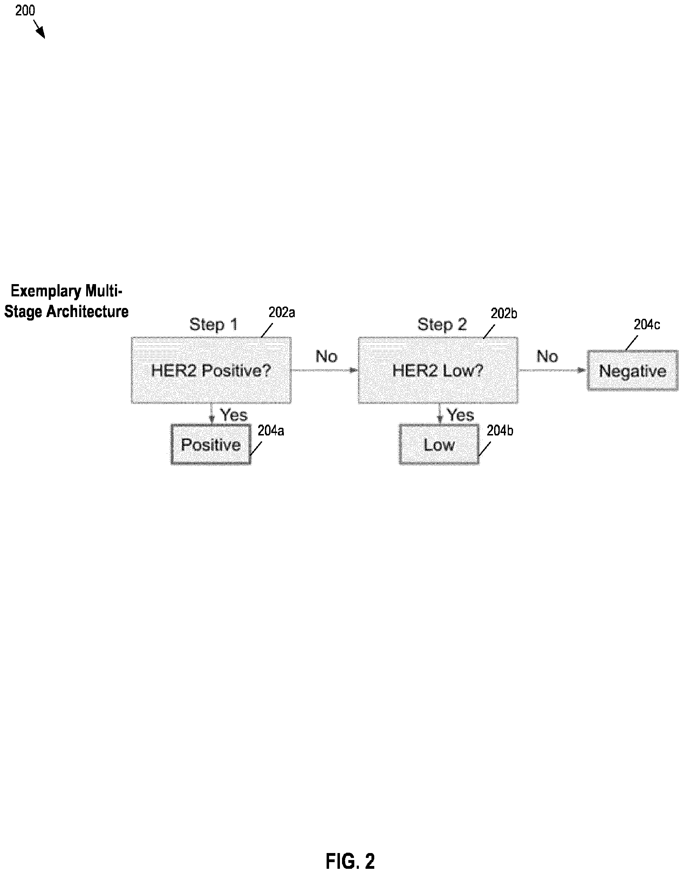

FIG. 2 depicts an exemplary multi-stage machine learning model architecture 200, according to some aspects. In some aspects, the model architecture 200 (e.g., a predictive/diagnostic model having two stages) may be trained on a feature space composed of only RNA features. In some aspects, the model architecture 200 (e.g., a multi-omic predictive/diagnostic model having two stages 202) may be based on a feature space composed of RNA features and copy number variant features. Each of the two stages 202 may include a respective model type (e.g., a respective random forest model trained on, respectively, CNV and RNA-based features), but the models in each stage 202 may be trained on different labels. Specifically, a first stage model 202a may be trained using labeled data having binary labels of (HER2-positive, NOT-HER2-positive). A second stage model 202b may be trained using labeled data having binary labels of (HER2-low, NOT-HER2-low).

For example, a library such as Scikit-learn, R random forest, Java Weka, etc. may be used to implement the random forest model training. RNA features used for training may correspond to one or more of'GRB7', 'GSDMB', 'MIEN1', 'ORMDL3', 'PGAP3', 'PSMD3', 'STARD3' and/or 'ERBB2' genes. As discussed, in some aspects, copy number variant scores or counts may be used as training features. Multiple copy number variant features across a plurality of genes may be used for training. For example, the copy number variant features used for training may correspond to one or more of 'BRD4', 'CDK12', 'ERBB2' and/or 'RARA' genes. In some aspects, more and/or different features may be used for training.

For RNA features, the present techniques may generate feature values for training that are log of transcripts per million of the RNA expression of genes. The feature values may be continuous, non-negative numbers. The corresponding training values may be the HER2 labels discussed above. The training labels may be determined using IHC and/or FISH. Thus, a model trained using this training data (e.g., the architecture 200) may learn to predict outputs that are consistent with IHC and FISH techniques for similar samples. IHC label values may be 0, 1, 2 or 3. FISH label values may be positive or negative. Generally, an IHC label value of 0 corresponds to an HER2-negative sample. An IHC label value of 1 corresponds to an HER2-low sample, and an IHC label value of 2 requires use of FISH to for further disambiguation. In this case, if FISH is positive, then the sample is HER2-positive. If FISH is negative, then the sample is HER2-low. An IHC label value of 3 indicates that the sample is HER2-positive. Training the machine learning models herein may include fitting the RNA expression values to the IHC/FISH label data using strategies including random forest algorithms.

For copy number variation training data, the present techniques may use an algorithm to generate a continuous number that represents an estimate of the number of copy number variations of a given sample, per gene. For example, the method described in U.S. Patent No. 11,705,226, entitled "Data based cancer research and treatment systems and methods," filed on October 18, 2019, hereby incorporated by reference in its entirety, for all purposes, may be used to generate the estimate of the number of copy number variations. Specifically, as described in the '226 patent, FIG. 313 describes a CNV pipeline that may be used for this purpose. Two features may be used, in some cases, to train the architecture 200: a major copy number feature and an afterwards copy number features. The major copy number feature may estimate the number of copies for a chromosome. The afterwards may be an estimate of the confidence in the major copy number. These two features may be used for each of the copy number variation genes used for training.

In operation, the model architecture 200 (i.e., the stage one model 202a and stage two model 202b) may assign labels to samples successively, in a cascade. In the first stage, the model 202a identifies HER2-positive samples (block 204a), and in the second stage, the model 202b identifies HER2-low samples (block 204b). The architecture 200 may annotate samples that are neither annotated as HER2-positive nor HER2-low as HER2-negative samples (block 204c). In each of these two steps, the model 200 may include one or more random forest models trained on RNA-based features, and optionally copy number variant features. The models 202 may be trained on different labels. Specifically, the first stage model 202a may be trained on data with (HER2-positive, NOT-HER2-positive) labels, whereas the second stage model 202b may be trained data labeled with (HER2-low, NOT-HER2-low) labels.

In some aspects, the architecture 200 may be trained only using primary cancer data, or metastatic cancer data. In some aspects, the architecture 200 may be trained to make predictions with respect to a specific cancer type, such as breast cancer. In some aspects, the architecture 200 may be trained for a subset of cancer types. The selection of different training strategies/ objectives may require different training sets to be configured. In general, empirical testing has shown high confidence in predictions for a model trained on data using a sample of around 900 patients.

It is envisioned that algorithms other than random forest may be used to train the architecture 200. For example, XGBoost, logistic regression and support vector machines are alternative algorithms to random forest.

In some aspects, the present techniques may be provided to another party, for example via a platform-as-a-service (PaaS) offering, software-as-a-service (SaaS), a white labeled service, etc. Such other parties may include pharmaceutical companies, biotechnical companies, contract research organizations, startups (e.g., personalized medicine firms), healthcare analytics companies, software companies, pharmacogenomics companies, healthcare consultancies, non-profit research organizations, etc.

In some examples, the HER2 status of a patient may be utilized to ascertain whether the patient satisfies the inclusion or exclusion criteria for a specific clinical trial. Alternatively, the HER2 statuses of numerous patients within a database may be employed to estimate the number of patients who might be suitable candidates for a particular trial. This approach may include processing molecular data, including RNA and potentially DNA data or copy number variant data, to predict the HER2 status of patients. For example, by employing a trained multi-stage machine learning architecture, the process may include determining if the molecular data indicates a HER2-positive status. If not HER2-positive, the data may be further analyzed to identify if the patient's status is HER2-low. In cases where the patient's status is neither HER2-positive nor HER2-low, the status may be designated as HER2-negative. This methodology enables the generation of a digital HER2-low status report for the patient, which can be displayed to inform clinical trial eligibility decisions. Utilizing molecular data for HER2 status prediction not only enhances the precision and reproducibility of HER2 status testing but also expands the potential patient population eligible for HER2-based targeted therapies by identifying patients with HER2-low status who may benefit from such treatments.

Specifically, as discussed, IHC/FISH is sometimes not run clinically due to insufficient tissue, low likelihood of positivity, or being stuck in old clinical practice patterns. The present machine learning-based molecular diagnostic testing enables expression calls for any patient receiving a next-generation sequencing report, even if the patient did not receive IHC or FISH testing. The assay can also be used in pharmaceutical applications for identification of HER2-low and HER2-positive patients using next-generation sequencing data only. The HER2-low patient population has come in the spotlight for development of targeted therapies, especially after the success of the Destiny-Breast 04 trial. The need to diagnose Ultra-low HER2 population will increase if the results from Destiny-Breast 06 trial are positive, and a molecular signature for diagnosing Ultra-low HER2 will be useful to find the minimum HER2 threshold that will still lead to a drug (e.g., Trastuzumab deruxtecan (TDxd)) response. There is also a possibility that molecular assays (including the present disclosure) will replace IHC/FISH assays for HER2-low diagnosis, in general, given the high variability of HER2-low predictions via IHC/FISH.

The architecture 200 may be trained to use a selected feature space (i.e., a signature) for predicting HER2 status. In general, the signature includes RNASeq and CNV genomic data across one or more genomic regions. In some cases, a subset of genes may be analyzed.

In some aspects, the machine learning architecture 200 may include multiple stages, each represented by a plurality of models 202, that make one or more respective predictions 204. The model may use variable screening, future screening, random forest trees and/or binary classifiers. For example, in the first stage 202a, the predictor model may identify HER2-positive samples (block 204a). In the second stage 202b, the predictor model may identify HER2-low samples (block 204b). Samples that are not HER2-positive or HER2-low may be classified as HER2-negative (block 204c). The model architecture 200 may begin without any indication of which features are relevant. The model architecture 200 may be trained by a process of eliminating irrelevant features. Feature screening may be suitable in high dimensional feature spaces to avoid the curse of dimensionality when performing model or variable selection. Thus, variable screening may be used before variable selection in the present techniques.

In the case of HER2 screening, HER2-positive sample identification at the first stage 202a of the model architecture 200 may be performed with significantly more relative confidence than HER2-low sample identification at the second stage 202b. This difference in confidence may be a function of the fact that HER2-low is a newer categorization that does not yet have rigorous clinical conventional methods. Thus, the present techniques may include eliminating possible results that are HER2-positive in the first stage 202a (e.g., using a binary classifier trained to identify a given sample as either corresponding or not corresponding to HER2-positive tissue), which is performed with relatively high confidence. In other words, irrelevant variables may be eliminated at the stage 202a and simplify the overall analysis. After detecting HER2-positives at block 204a, the architecture 200 may proceed to the second stage 202b (e.g., using a binary classifier trained to identify a given sample as either corresponding to or not corresponding to HER2-low tissue).

In one embodiment, an advantageous benefit of the cascaded model architecture 200 (i.e., a multi-stage model) is that once the HER2-positive data is removed at the stage 202a (block 204a), the biological data set becomes much smaller and more manageable. In the stage 202b, the question is how many of the remaining samples are HER2-low. The stage 202b may include a second trained machine learning model that annotates sample data as corresponding to (or not corresponding to) HER2-low negative samples, and annotates everything else as HER2-negative.

In one embodiment, a significant benefit of the present multi-stage cascaded model architecture 200 is that because the model of the first stage 202a may be trained to detect HER2-negative samples, and the second model at the second stage 202b may be trained to detect HER2-low samples, no model need be trained to identify HER2-negative samples. This is a significant savings in computational effort/work, because any given HER2-negative sample might correspond to any of a number of different types of cancer, including breast cancer HER2-negative cancer sample, a primary HER2-negative cancer sample, a metastatic HER2-negative cancer sample, a colorectal HER2-negative cancer sample, a pancreatic HER2-negative cancer sample, a bladder HER2-negative cancer sample, etc. The list of potential HER2-negative cancers is long. HER2-positive and HER2-low cancer samples are a much smaller class of possible sample outcomes, so a significant majority of the complexity of screening for HER2-negative cancer samples (a much larger class of potential classification outcomes) is avoided.

While the present techniques describe a multi-stage model architecture 200 that is inherently optimized for processing RNASeq data, due to its cascaded structure, it is envisioned that other less efficient architectures may nonetheless be suitable for solving the HER2-low classification problem. For example, modifying the training and/or inference data types may give rise to more suitable architectures. For example, in some cases, the second stage 202b could be configured to be a tumor-agnostic diagnostic machine learning model, in which multiple sub-models are configured to perform classification. For example, the second stage 202b could be composed of a trained model for breast cancer, a trained model for pancreatic cancer, a trained model for bladder cancer, and so on. The amount and type of data may dictate such architectural decisions. It is also appreciated that increasing the number of predictive models might reduce bias, but would likely require more data (i.e., a bias-variance tradeoff).

### Exemplary Computing Environments

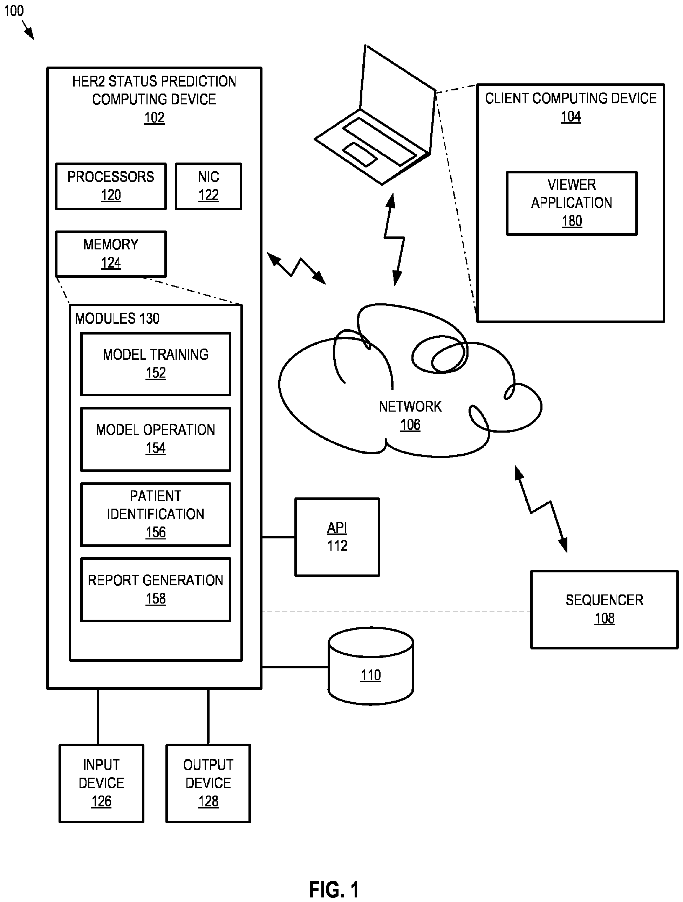

FIG. 1 illustrates an exemplary computing environment 100 for performing the present techniques, according to some aspects. The environment 100 may include computing resources for determining HER2 status of a patient, and/or for further processing/ computations based on such predictions, such as patient identification/notification and report generation.

The computing environment 100 may include a HER2 status prediction computing device 102, a client computing device 104, an electronic network 106, a sequencer system 108 and an electronic database 110. The HER2 status prediction computing device 102 may include an application programming interface 112 that enables programmatic access to the HER2 status prediction computing device 102. The components of the computing environment 100 may be communicatively connected to one another via the electronic network 106, in some aspects. Each will now be described in greater detail.

The HER2 status prediction computing device 102 may implement, *inter alia,* training and operation of machine learning models for predicting HER2 status of one or more patients, patient identification and report generation. In some aspects, the HER2 status prediction computing device 102 may be implemented as one or more computing devices (e.g., one or more servers, one or more laptops, one or more mobile computing devices, one or more tablets, one or more wearable devices, one or more cloud-computing virtual instances, etc.). The HER2 status prediction computing device 102 may include one or more processors 120, one or more network interface controllers 122, one or more memories 124, an input device 126 and an output device 128.

In some aspects, the one or more processors 120 may include one or more central processing units, one or more graphics processing units, one or more field-programmable gate arrays, one or more application-specific integrated circuits, one or more tensor processing units, one or more digital signal processors, one or more neural processing units, one or more RISC-V processors, one or more coprocessors, one or more specialized processors/accelerators for artificial intelligence or machine learning-specific applications, one or more microcontrollers, etc.

The HER2 status prediction computing device 102 may include one or more network interface controllers 122, such as Ethernet network interface controllers, wireless network interface controllers, etc. The network interface controllers 122 may include advanced features, in some aspects, such as hardware acceleration, specialized networking protocols, etc.

The memories 124 of the HER2 status prediction computing device 102 may include volatile and/or non-volatile storage media. For example, the memories 124 may include one or more random access memories, one or more read-only memories, one or more cache memories, one or more hard disk drives, one or more solid-state drives, one or more non-volatile memory express, one or more optical drives, one or more universal serial bus flash drives, one or more external hard drives, one or more network-attached storage devices, one or more cloud storage instances, one or more tape drives, etc.

The memories 124 may have stored thereon one or more modules 130, for example, as one or more sets of computer-executable instructions. In some aspects, the modules 130 may include additional storage, such as one or more operating systems (e.g., Microsoft Windows, GNU/Linux, Mac OSX, etc.). The operating systems may be configured to run the modules 130 during operation of the HER2 status prediction computing device 102 - for example, the modules 130 may include additional modules and/or services for receiving and processing quantitative data. The modules 130 may be implemented using any suitable computer programming language(s) (e.g., Python, JavaScript, C, C++, Rust, C#, Swift, Java, Go, LISP, Ruby, Fortran, etc.). The memories may be non-transitory memories.

The modules 130 may include a machine learning model training module 152, a model operation module 154, a patient identification module 156 and a report generation module 158. In some aspects, more or fewer modules 130 may be included. The modules 130 may be configured to communicate with one other (e.g., via inter-process communication, via a bus, message queue, sockets, etc.).

The machine learning model training module 152 may include sets of computer-executable instructions for training one or more machine learning models based on training data. The machine learning model training module 152 may take input data, often in the form of a dataset, and use it to train a machine learning model. The machine learning model training module 152 may prepare the input data by performing data cleaning, feature engineering, data splitting (into training and validation sets), and handling missing values or outliers. The machine learning model training module 152 may select a machine learning algorithm or model architecture to use for the task at hand. Specifically, the machine learning model training module 152 may include sets of computer-executable instructions for implementing machine learning training architectures such as the cascaded model architecture 200 of FIG. 2. The machine learning model training module 152 may select a random forest algorithm for or more stages of the model architecture 200.

The machine learning model training module 152 may include instructions for performing hyperparameter tuning (e.g., settings or configurations for the model that are not learned from the data but need to be specified before training). The machine learning model training module 152 may use grid search or other techniques for specifying hyperparameters. Examples of hyperparameters that may be selected for random forest algorithms include the number of decision trees in the forest, the maximum depth of each individual decision tree in the forest, the minimum number of samples required to split a node in a tree, the minimum number of samples required to be in a leaf node, the maximum number of features to consider for splitting, and a bootstrapping parameter.

The machine learning model training module 152 may include instructions for training the selected machine learning model on the training data. The training process involves optimizing the model's parameters to make predictions. In the case of a random forest algorithm, a voting mechanism (e.g., majority vote) may be used to determine a final prediction. After training, the machine learning model training module may evaluate the trained model's performance using validation data. For random forest models, validation techniques may include out-of-bag validation and cross-validation.

The machine learning model training module 152 may include instructions for serializing and deserializing stored models. This enables the machine learning model training module 152 to store the trained model as data, and to reload the model and use the trained model for prediction without retraining the model.

The model operation module 154 may include computer-executable instructions for operating one or more trained machine learning models. For example, the model operation module 154 may have access to next-generation sequencing data via the sequencer 108, in some aspects. The model operation module 154 may load one or more model trained by the model training module 152. The model operation module 154 may receive raw next generation sequencing data, preprocess it, apply one or more trained machine learning models, and generate one or more predictions (e.g., HER2 status, specifically HER2-low status) corresponding to the raw next generation sequencing data.

The model operation module 154 may receive next-generation sequencing data including DNA sequencing data (e.g., whole-genome sequencing, exome sequencing), RNA sequencing data (RNAseq), ChIP sequencing data (ChIP-seq), etc. The model operation module 154 may include instructions for receiving and processing data encoded in multiple different formats (FASTQ, BAM, VCF, etc.).

The model operation module 154 may perform quality control, read alignment, variant calling, and data normalization.

The model operation module 154 may perform feature engineering to transform raw sequencing data into features that can be used by one or more machine learning models trained by the machine learning training module 152.

The model operation module 154 may process the received sequencing data using one or more trained machine models (e.g., a random forest model, a deep learning model, support vector machine, etc.) to preprocessed sequencing data. The model operation module 154 may generate model performance statistics, such as accuracy, precision, recall, F1-score, or AUC-ROC for classification techniques.

The patient identification module 156 may include instructions for identifying one or more patients based on outputs of the machine learning operation module 154. Specifically, patients may opt in to receive identification notices based on determinations of their specific HER2 status, and in particular, on their HER2-low status. As discussed above, HER2-low status has been shown to be relevant for targeted therapies. However, identifying patients has been conventionally very difficult. By performing analysis of a patient's specific tumor profile, the present techniques can rigorously identify patients who may benefit from such targeted treatments, without the biases inherent to traditional techniques. This enables precision medicine to be provided to patients in a manner that improves the field of machine learning-based cancer therapies. Further, the patient identification module 156 may identify patients that may benefit from research or clinical trials. Thus, in some aspects, the patient identification module 156 may include patient notification instructions that are configured to automatically notify a patient of clinical trial or targeted therapy eligibility or matches based on the output of the machine learning analysis.

Herein, a targeted therapy matched by the machine learning analysis may include a systemic therapy, an external beam radiation therapy, a surgical therapy, an observation therapy, an ADC (antibody drug conjugate) therapy targeting HER2, etc.

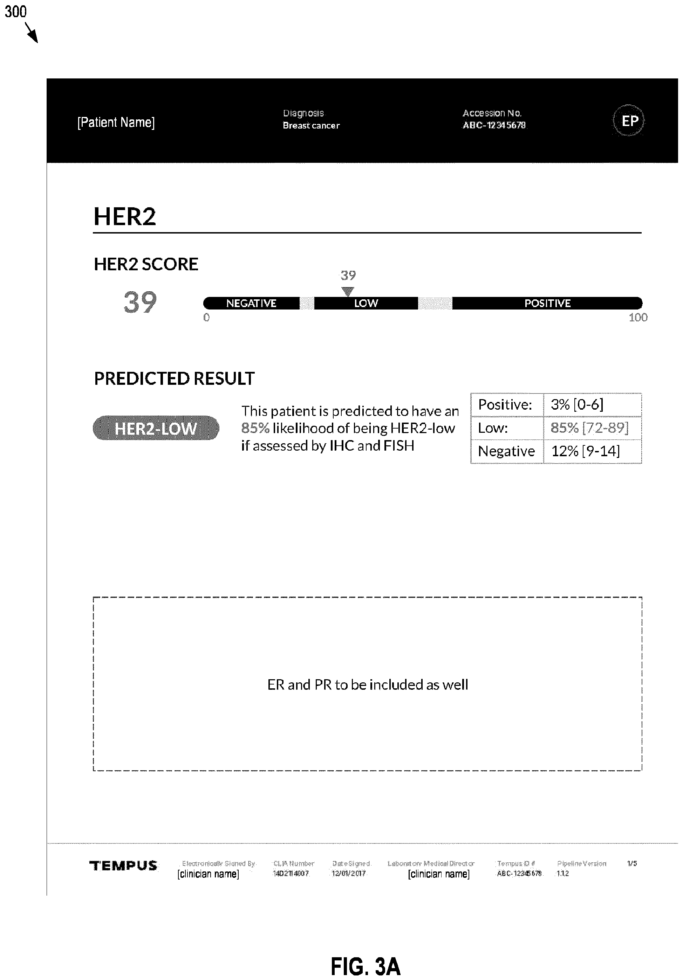

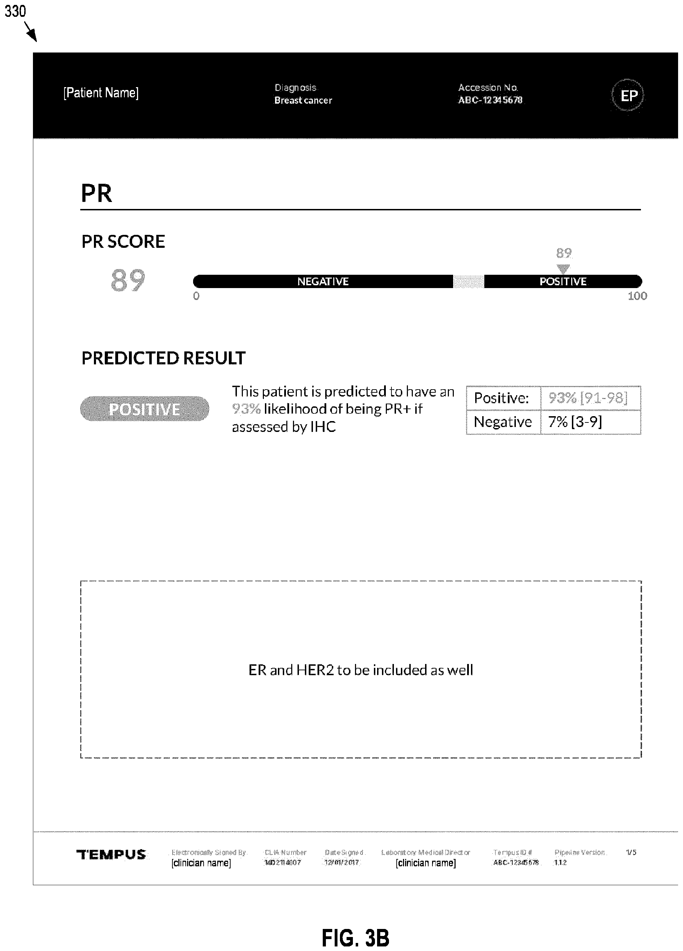

In some aspects, the report generation module 158 may generate reports that include predictions regarding the confidence of classifications of patient data (for example, the HER2 classification result of the model disclosed here), as shown in FIG. 3A and FIG. 3B. For example, these reports may take the form of text documents, digital presentations, word processing documents, etc. For example, the report generation module 158 may generate one or more reports that include a HER2 score of a patient. The reports may include stratified likelihoods that the patient has an HER2-low status, as would be measured by IHC and/or FISH. The report generation module 158 may also generate, based on outputs generated by one or more trained model, progesterone receptor positive (PR+) status and estrogen receptor (ER) statistics and/or predictions. These results enable clear and actionable predictions, especially by providing an improvement to clinicians by providing HER2, PR, and ER biomarkers in a single predictive system. This improves upon systems that do not include each biomarker, and helps clinicians to make better treatment decisions, and helps automated systems to provide better treatment matches.

The report generation module 158 may include computer-executable instructions for generating machine-readable results. For example, the client computing device 104 may be accessed by a user to view results generated by the prediction computing device 102. For example, a user may access a mobile device, laptop device, thin client, etc. embodied as the client computing device 104 to view simulation and confidence scoring results, and/or reports, with respect to a sample whose values were processed by the prediction computing device 102. Information from the prediction computing device 102 may be transmitted via the network 106 (e.g., for display via the viewer application 180).

The electronic network 106 may communicatively couple the elements of the environment 100. The network 106 may include public network(s) such as the Internet, a private network such as a research institution or corporation private network, and/or any combination thereof. The network 106 may include a local area network (LAN), a wide area network (WAN), a cellular network, a satellite network, and/or other network infrastructure, whether wireless or wired.

In some aspects, the network 106 may be communicatively coupled to and/or part of a cloud-based platform (e.g., a cloud computing infrastructure). The network 106 may utilize communications protocols, including packet-based and/or datagram-based protocols such as Internet protocol, transmission control protocol, user datagram protocol, and/or other types of protocols. The network 106 may include one or more devices that facilitate network communications and/or form a hardware basis for the networks, such as one or more switches, one or more routers, one or more gateways, one or more access points (such as a wireless access point), one or more firewalls, one or more base stations, one or more repeaters, one or more backbone devices, etc.

The sequencer system 108 may include a next generation sequencer, such as an RNA sequencing whole exome capture transcriptome assay and a DNA sequencing assay that may be whole genome sequencing or a targeted panel (for example, a targeted oncology panel using hybrid-capture library preparation).

The electronic database 110 may include one or more suitable electronic databases for storing and retrieving data, such as relational database (e.g., MySQL databases, Oracle databases, Microsoft SQL Server databases, PostgreSQL databases, etc.). The electronic database 110 may be a NoSQL database, such as a key-value store, a graph database, a document store, etc. The electronic database 110 may be an object-oriented database, a hierarchical database, a spatial database, a time-series database, an in-memory database, etc. In some aspects, some or all of the electronic database 110 may be distributed.

In operation, one or more sequencer runs may be performed using the sequencer 108, either by the company operating/ controlling the environment 100 or by another party. The results of the sequencer may be received by the HER2 status prediction computing device 102 as sequencer data. The HER2 status prediction computing device 102 may preprocess the sequencer data, optionally storing some or all of it in the electronic database 110. of FIG. 1. The HER2 status prediction computing device 102 may load one or more trained models and provide the sequencer data as input to the one or more trained models, and receive predictions as to HER2 status from the one or more trained models. In some aspects, the HER2 status prediction computing device 102 may receive DNA data in addition to the sequencer data, such as copy number variation data. The HER2 status prediction computing device 102 may provide both of the sequencer data (e.g., RNASeq data) and copy number variation data to the trained machine learning model as a molecular signature. The HER2 status prediction computing device 102 may generate a prediction as to the samples corresponding to the RNASeq and copy number variation data.

In some aspects, the database 110 may include additional clinical and molecular patient data. For example, the database 110 may include qualitative insights into patient history, symptoms, and clinical reasoning behind treatment decisions, offering a narrative context to the quantitative data also stored within the database. The database 110 may include lab results and test results, ranging from basic blood tests, imaging, analyses of the imaging, to more complex genetic screenings. The database 110 may store detailed diagnoses, and DNA and RNA sequencing data. In some aspects, the database 110 may include methylation assay results, or other information related to epigenetic modifications or disease etiology. In some aspects, the database 110 may include therapy response data or other tracking information related to how patients respond to various treatments. The database 110 may also incorporate results from the methods described in the application, such as risk profiles or HER2 status predictions generated through the application's trained multi-stage machine learning architecture. By integrating these results, the database enhances the utility of molecular data in clinical decision-making, enabling healthcare providers to identify patients who may benefit from targeted therapies based on their HER2 status. This capability represents a significant advancement in the field of oncology, where HER2 status is an important factor in determining the most appropriate treatment for cancer patients.

By the time that the HER2 status prediction computing device 102 uses the trained models in this way, the trained models have already been trained using a training data set as described above, wherein one or more sub-models that are part of the model architecture (e.g., the model architecture 200) are individually trained to perform binary classification using labeled data sets of RNASeq data and copy number variation data, respectively. In some aspects, as mentioned, other data such as proteomics data may be used for training and inference.

Predictions of the HER2 status prediction computing device 102 may be stored, for example in the memory 124 or the database 110. These results may be provided directly to other elements of the environment 100, for example, via the network 100. These results may also be processed further, for example, to identify/notify patients via the patient identification module 156 and/or to generate digital reports by the report generation module 158.

### Exemplary Computer-Implemented Digital Reports

FIG. 3A depicts an exemplary digital report 300 generated by the elements of the environment 100, according to some aspects. For example, the report 300 may be generated by the report generation module 158, and displayed via the output device 128 (e.g., via computer monitor, a laptop screen, etc.) and/or via the viewer application 180 of the client computing device 104. The FIG. 3A generally depicts a digital report that may be provided to a patient (e.g., via email) that includes the patient's HER2 status, as predicted by one or more trained machine learning models (e.g., the model architecture 200 of FIG. 2). The report 300 may include percentage likelihoods of the patient being HER2-low if assessed by IHC/FISH, as well as the percentage likelihood of the patient being HER2-negative and/or HER2-positive. In some aspects, the model computes percentage likelihood is computed by comparing the patient's RNA and CNV data to the positive and negative controls to determine a numeric estimate of the patient's similarity to the positive controls and negative controls, respectively.

The report 300 may include one or more diagnoses (e.g., of cancers such as breast cancer). The report 300 may include additional diagnostics, such as estrogen receptor status.

FIG. 3B depicts an exemplary digital report 330 of a patient's progesterone receptor status, according to some aspects. The report 330 may include a PR score, that reflects a scaled result corresponding to progesterone receptor status. The report 330 may further include percentage likelihoods of the patient being PR negative or positive, as determined by one or more trained machine learning models.

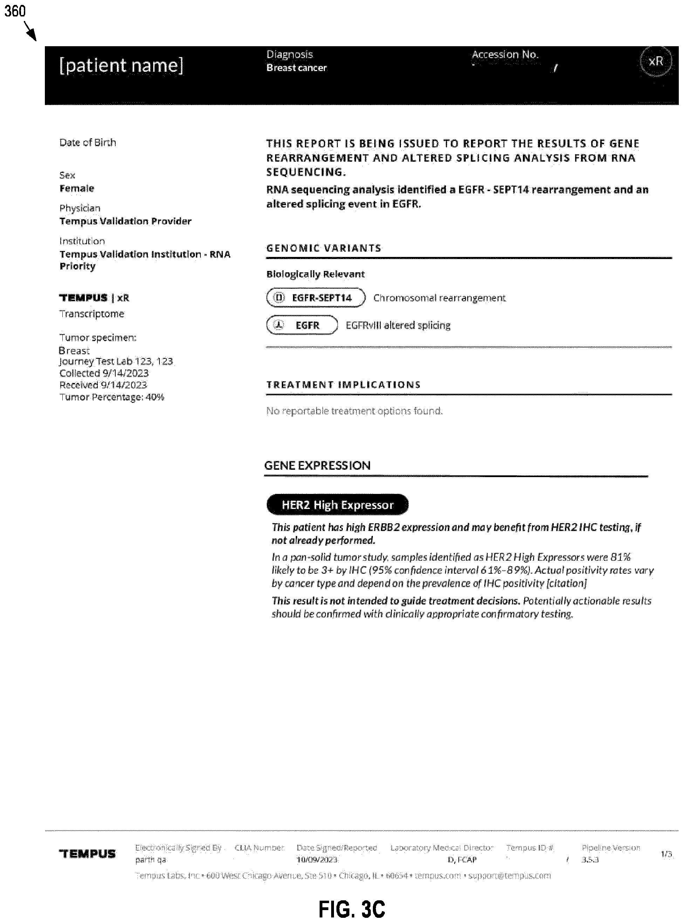

FIG. 3C depicts an exemplary digital report of a patient's gene rearrangement and altered splicing analysis from RNA sequencing, according to some aspects. The report includes the patient's diagnosis of breast cancer and contains several sections detailing the results and their potential clinical implications.

The top portion of the report provides the patient's name (redacted), the diagnosis of breast cancer, the accession number (not visible), date of birth (not visible), sex (Female), the sample type (Tumor specimen: Breast), physician's name (example text entered, Validation Provider), and institution (example text entered, Validation Institution - RNA Priority). It also displays the address of the laboratory where the test was conducted (example text entered, Journey Test Lab 123, 123), the collection date (9/14/2023), and the tumor percentage (40%).

Under the "GENOMIC VARIANTS" section, two findings are reported: "Biologically Relevant" with notations of "EGFR-SEPT14 Chromosomal rearrangement" and "EGFR EGFRvIII altered splicing". The "TREATMENT IMPLICATIONS" section states "No reportable treatment options found." The "GENE EXPRESSION" section contains a statement regarding the HER2 High Expressor status of the patient: "This patient has high ERBB2 expression and may benefit from HER2 IHC testing, if not already performed. In a non-solid tumor study, samples identified as HER2 High Expressors were 81% likely to be 3+ by IHC (95% confidence interval of 61%-89%). Actual positivity rates vary by cancer type and depend on the prevalence of HER2 positivity (citation)." It also issues a caution that "This result is not intended to guide treatment decisions. Potentially actionable results should be confirmed with clinically appropriate confirmatory testing."

The bottom of the report includes an electronic signature by the pathologist, the CLIA number, the date when the report was signed/reported (not visible), the laboratory medical director, the laboratory address (Tempus Labs, Inc. • 600 West Chicago Avenue, Ste 510 • Chicago, IL • 60654 • tempus.com • support@tempus.com), an identifier ID, and a Pipeline Version (3.5.3).

### Exemplary Computer-Implemented Methods

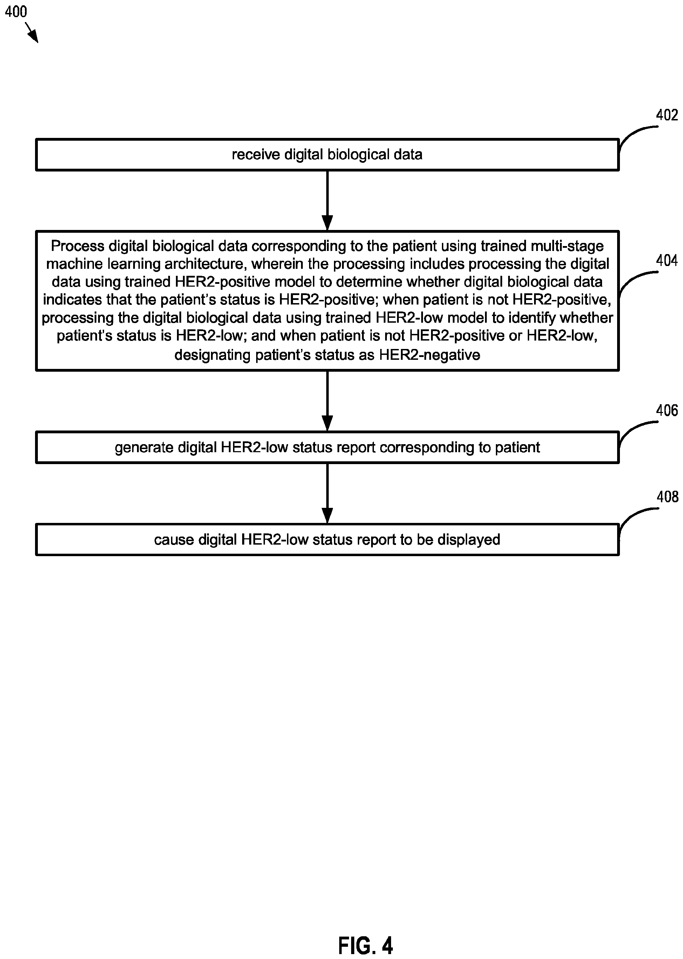

FIG. 4 depicts an exemplary flow diagram of a computer-implemented method 400 for determining HER2-low status of a patient using molecular data of the patient, according to some aspects. The method 400 may be implemented using the environment 100 of FIG. 1.

The method 400 may include receiving, via one or more processors, digital biological data (block 402).

The method 400 may include processing, via one or more processors, the digital biological data corresponding to the patient using a trained multi-stage machine learning architecture, wherein the processing includes processing the digital data using a trained HER2-positive model to determine whether the digital biological data indicates that the patient's status is HER2-positive; when the patient is not HER2-positive, processing the digital biological data using a trained HER2-low model to identify whether the patient's status is HER2-low; and when the patient is not HER2-positive or HER2-low, designating the patient's status as HER2-negative (block 404). The method 400 may include generating, via one or more processors, a digital HER2-low status report corresponding to the patient (block 406). The method 400 may include causing, via a display device, the digital HER2-low status report to be displayed (block 408). In some aspects, the digital biological data includes RNA data. In some aspects, the digital biological data includes at least some transcriptomic data. In some aspects, the at least some of the transcriptomic data includes at least some data generated via RNA seq. In some aspects, the digital biological data includes at least one of DNA data or copy number variant data. In some aspects, receiving the digital biological data includes receiving the digital biological data from a next-generation sequencing platform. In some aspects, the trained HER2-positive model is a random forest model. In some aspects, the trained HER2-low model is a random forest model. In some aspects, the trained HER2-positive model is a binary classifier trained on molecular signature data labeled according to (HER2-positive, NOT-HER2-positive) labels. In some aspects, the trained HER2-low model is a binary classifier trained on molecular signature data labeled according to (HER2-low, NOT-HER2-low) labels.

In some aspects, the method 400 may include generating a prediction as to the HER2-low, HER2-positive and/or HER2-negative status of a given sample based on the trained multi-stage machine learning architecture. In some aspects, the method 400 may include identifying at least one patient from a population of patients by processing the data of the patient using trained multi-stage machine learning architecture; and matching the identified patient for treatment using a targeted therapy. In some aspects, the targeted therapy is a HER2 targeted therapy. In some aspects, the targeted therapy is trastuzumab deruxtecan.

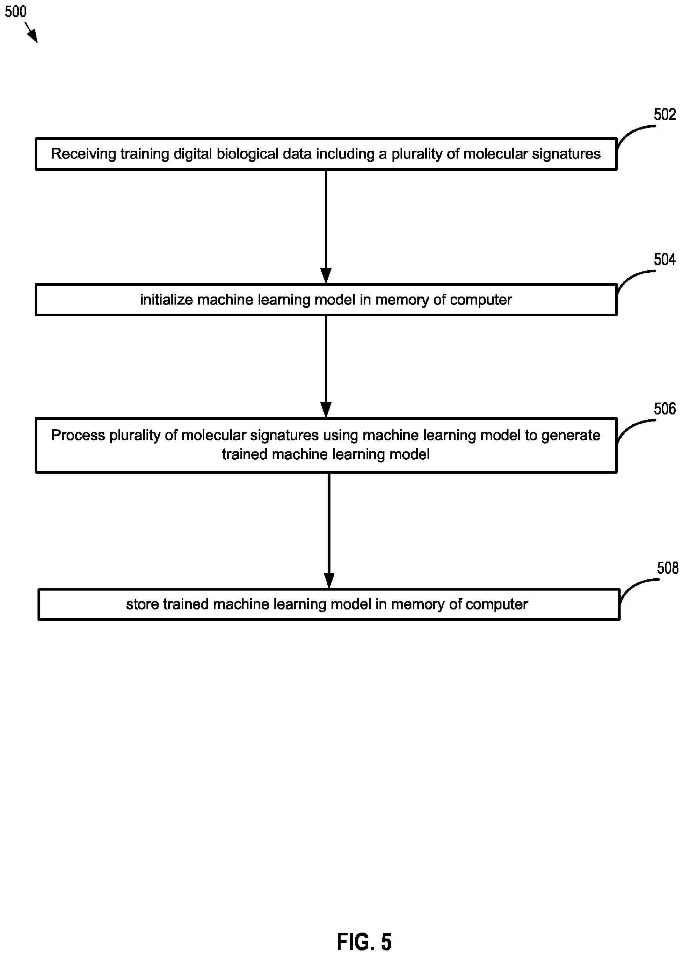

FIG. 5 depicts a computer-implemented method 500 for training a model architecture to determine HER2-low status of a patient using molecular data of the patient. The method 500 may include receiving, via one or more processors, training digital biological data, the training digital biological data including a plurality of molecular signatures, each having a respective label (block 502). The method 500 may include initializing, via one or more processors, a machine learning model in a memory of a computer, the machine learning model having a plurality of hyperparameters (block 504). The method 500 may include processing, via one or more processors, the plurality of molecular signatures in the training digital biological data and the respective label of each of the plurality of molecular signatures using the machine learning model to generate a trained machine learning model (block 506). The method 500 may include storing, via one or more processors, the trained machine learning model in a memory of a computer, wherein the storing includes generating a serialized copy of the machine learning model, and writing the serialized copy of the machine learning model to the memory of the computer (block 508).

In some aspects, the computer-implemented method 500 includes loading, via one or more processors, the serialized copy of the trained machine learning into a memory of a computer; generating an in-memory instantiation of the trained machine learning model by deserializing the serialized copy of the trained machine learning model; and performing the method of claim 1 using the in-memory instantiation of the trained machine learning model.

In some aspects, the training digital biological data includes at least one of RNASeq data, copy number variant data. In some aspects, the method 500 includes training the machine learning model using RNA features of at least one of'GRB7', 'GSDMB', 'MIEN1', 'ORMDL3', 'PGAP3', 'PSMD3', 'STARD3' or'ERBB2' genes. In some aspects, the method 500 includes training the machine learning model using copy number variant features of at least one of 'BRD4', 'CDK12', 'ERBB2' or 'RARA' genes. In some aspects the copy number variant data includes a respective estimated copy number variant and a respective estimated confidence of the estimated copy number variant, for each copy number variant. In some aspects, the machine learning model is a random forest model. In some aspects, processing the plurality of the molecular signatures in the training digital biological data and the respective label of the each of the plurality of the molecular signatures using the machine learning model to generate the trained machine learning model includes: training a first binary classifier to predict HER2-positive status; and training a second binary classifier to predict HER2-low status.

In some aspects, the method 500 includes generating a prediction as to the HER2-low, HER2-positive and/or HER2-negative status of a given sample based on the trained machine learning model. In some aspects, the method 500 includes identifying at least one patient from a population of patients by processing the patient's data using the trained machine learning model; and matching the identified patient for treatment using a targeted therapy. In some aspects, the targeted therapy is trastuzumab deruxtecan.

The various embodiments described above can be combined to provide further embodiments. All U.S. patents, U.S. patent application publications, U.S. patent application, foreign patents, foreign patent application and non-patent publications referred to in this specification and/or listed in the Application Data Sheet are incorporated herein by reference, in their entirety. Aspects of the embodiments can be modified if necessary to employ concepts of the various patents, applications, and publications to provide yet further embodiments.

These and other changes can be made to the embodiments in light of the above-detailed description. In general, in the following claims, the terms used should not be construed to limit the claims to the specific embodiments disclosed in the specification and the claims but should be construed to include all possible embodiments along with the full scope of equivalents to which such claims are entitled. Accordingly, the claims are not limited by the disclosure.

Aspects of the techniques described in the present disclosure may include any of the following aspects, either alone or in combination:
1. A computer-implemented method for determining HER2-low status of a patient using molecular data of the patient, comprising: receiving, via one or more processors, digital biological data; processing, via one or more processors, the digital biological data corresponding to the patient using a trained multi-stage machine learning architecture, wherein the processing includes: (i) processing the digital biological data using a trained HER2-positive model to determine whether the digital biological data indicates that a HER2 status of the patient is HER2-positive; (ii) when the HER2 status of the patient is not HER2-positive, processing the digital biological data using a trained HER2-low model to identify whether the HER2 status of the patient is HER2-low; and (iii) when the HER2 status of the patient is not HER2-positive or HER2-low, designating the HER2 status of the patient as HER2-negative; generating, via one or more processors, a digital HER2-low status report corresponding to the patient; and causing, via a display device, the digital HER2-low status report to be displayed.
2. The computer-implemented method of aspect 1, wherein the digital biological data includes RNA data.
3. The computer-implemented method of any of aspects 1-2, wherein the digital biological data includes at least some transcriptomic data.
4. The computer-implemented method of aspect 3, wherein the at least some of the transcriptomic data includes at least some data generated via RNA seq.
5. The computer-implemented method of any of aspects 1-4, wherein the digital biological data includes at least one of DNA data or copy number variant data.
6. The computer-implemented method of any of aspects 1-5, wherein receiving the digital biological data includes receiving the digital biological data from a next-generation sequencing platform.
7. The computer-implemented method of any of aspects 1-6, wherein the trained HER2-positive model is a random forest model.
8. The computer-implemented method of any of aspects 1-7, wherein the trained HER2-low model is a random forest model.
9. The computer-implemented method of any of aspects 1-8, wherein the trained HER2-positive model is a binary classifier trained on molecular signature data labeled according to (HER2-positive, NOT-HER2-positive) labels.
10. The computer-implemented method of any of aspects 1-9, wherein the trained HER2-low model is a binary classifier trained on molecular signature data labeled according to (HER2-low, NOT-HER2-low) labels.
11. The computer-implemented method of any of aspects 1-10, further comprising:
   generating a prediction as to the HER2-low, HER2-positive and/or HER2-negative status of a given sample based on the trained multi-stage machine learning architecture.
12. The computer-implemented method of any of aspects 1-11, further comprising: identifying at least one patient from a population of patients by processing the data of the patient using trained multi-stage machine learning architecture; and matching the identified patient for treatment using a targeted therapy.
13. The computer-implemented method of aspect 12, wherein the targeted therapy is a HER2 targeted therapy.
14. The computer-implemented method of any of aspects 11-13, wherein the targeted therapy is trastuzumab deruxtecan.
15. A computer-implemented method for training a model architecture to determine HER2-low status of a patient using molecular data of the patient, comprising: receiving, via one or more processors, training digital biological data, the training digital biological data including a plurality of molecular signatures, each having a respective label; initializing, via one or more processors, a machine learning model in a memory of a computer, the machine learning model having a plurality of hyperparameters; processing, via one or more processors, the plurality of molecular signatures in the training digital biological data and the respective label of each of the plurality of molecular signatures using the machine learning model to generate a trained machine learning model; and storing, via one or more processors, the trained machine learning model in a memory of a computer, wherein the storing includes generating a serialized copy of the machine learning model, and writing the serialized copy of the machine learning model to the memory of the computer.
16. The computer-implemented method of aspect 15, further comprising: loading, via one or more processors, the serialized copy of the trained machine learning into a memory of a computer; generating an in-memory instantiation of the trained machine learning model by deserializing the serialized copy of the trained machine learning model; and performing the method of aspect 1 using the in-memory instantiation of the trained machine learning model.
17. The computer-implemented method of any of aspects 15-16, wherein the training digital biological data includes at least one of RNASeq data, copy number variant data.
18. The computer-implemented method of aspect 17, further comprising: training the machine learning model using RNA features of at least one of'GRB7', 'GSDMB', 'MIEN1', 'ORMDL3', 'PGAP3', 'PSMD3', 'STARD3' or'ERBB2' genes.
19. The computer-implemented method of any of aspects 17-18, further comprising: training the machine learning model using copy number variant features of at least one of 'BRD4', 'CDK12', 'ERBB2' or 'RARA' genes.
20. The computer-implemented method of any of aspects 17-19, wherein the copy number variant data includes a respective estimated copy number variant and a respective estimated confidence of the estimated copy number variant, for each copy number variant.
21. The computer-implemented method of any of aspects 15-20, wherein the machine learning model is a random forest model.
22. The computer-implemented method of any of aspects 15-20, wherein processing the plurality of the molecular signatures in the training digital biological data and the respective label of the each of the plurality of the molecular signatures using the machine learning model to generate the trained machine learning model includes: training a first binary classifier to predict HER2-positive status; and training a second binary classifier to predict HER2-low status.
23. The computer-implemented method of aspect any of aspects 15-22, further comprising: generating a prediction as to the HER2-low, HER2-positive and/or HER2-negative status of a given sample based on the trained machine learning model.
24. The computer-implemented method of any of aspects 15-23, further comprising: identifying at least one patient from a population of patients by processing the patient's data using the trained machine learning model; and matching the identified patient for treatment using a targeted therapy.
25. The computer-implemented method of aspect any of aspects 15-24, wherein the targeted therapy is trastuzumab deruxtecan.
26. A computing system comprising: one or more processors; and one or more memories having stored thereon computer-readable instructions that, when executed, cause the computing system to: receive digital biological data; process the digital biological data corresponding to a patient using a trained multi-stage machine learning architecture, wherein the processing includes: (i) processing the digital data using a trained HER2-positive model to determine whether the digital biological data indicates that a HER2 status of the patient is HER2-positive; (ii) when the patient is not HER2-positive, processing the digital biological data using a trained HER2-low model to identify whether the HER2 status of the patient is HER2-low; and (iii) when the patient is not HER2-positive or HER2-low, designating the HER2 status of the patient as HER2-negative; generate, via one or more processors, a digital HER2-low status report corresponding to the patient; and causing, via a display device, the digital HER2-low status report to be displayed.
27. A computer-readable medium having stored thereon computer-executable instructions that, when executed, cause a computer to: receive digital biological data; process the digital biological data corresponding to a patient using a trained multi-stage machine learning architecture, wherein the processing includes: (i) processing the digital data using a trained HER2-positive model to determine whether the digital biological data indicates that a HER2 status of the patient is HER2-positive; (ii) when the patient is not HER2-positive, processing the digital biological data using a trained HER2-low model to identify whether a HER2 status of the patient is HER2-low; and (iii) when the patient is not HER2-positive or HER2-low, designating the HER2 status of the patient as HER2-negative; generate, via one or more processors, a digital HER2-low status report corresponding to the patient; and cause, via a display device, the digital HER2-low status report to be displayed.
28. A computing system comprising: one or more processors; and one or more memories having stored thereon computer-readable instructions that, when executed, cause the computing system to: receive training digital biological data, the training digital biological data including a plurality of molecular signatures, each having a respective label; initialize a machine learning model in a memory of a computer, the machine learning model having a plurality of hyperparameters; process the plurality of molecular signatures in the training digital biological data and the respective label of each of the plurality of molecular signatures using the machine learning model to generate a trained machine learning model; and store the trained machine learning model in a memory of a computer, wherein the storing includes generating a serialized copy of the machine learning model, and writing the serialized copy of the machine learning model to the memory of the computer.
29. A computer-readable medium having stored thereon computer-executable instructions that, when executed, cause a computer to: receive training digital biological data, the training digital biological data including a plurality of molecular signatures, each having a respective label; initialize a machine learning model in a memory of a computer, the machine learning model having a plurality of hyperparameters; process the plurality of molecular signatures in the training digital biological data and the respective label of each of the plurality of molecular signatures using the machine learning model to generate a trained machine learning model; and store the trained machine learning model in a memory of a computer, wherein the storing includes generating a serialized copy of the machine learning model, and writing the serialized copy of the machine learning model to the memory of the computer.

### ADDITIONAL CONSIDERATIONS

The computer-readable media may include executable computer-readable code stored thereon for programming a computer (e.g., comprising a processor(s) and GPU(s)) to the techniques herein. Examples of such computer-readable storage media include a hard disk, a CD-ROM, digital versatile disks (DVDs), an optical storage device, a magnetic storage device, a ROM (Read Only Memory), a PROM (Programmable Read Only Memory), an EPROM (Erasable Programmable Read Only Memory), an EEPROM (Electrically Erasable Programmable Read Only Memory) and a Flash memory. More generally, the processing units of the computing device 1300 may represent a CPU-type processing unit, a GPU-type processing unit, a TPU-type processing unit, a field-programmable gate array (FPGA), another class of digital signal processor (DSP), or other hardware logic components that can be driven by a CPU.

A system for performing the methods described herein may include a computing device, and more particularly may be implemented on one or more processing units, for example, Central Processing Units (CPUs), and/or on one or more or Graphical Processing Units (GPUs), including clusters of CPUs and/or GPUs. Features and functions described may be stored on and implemented from one or more non-transitory computer-readable media of the computing device. The computer-readable media may include, for example, an operating system and software modules, or "engines," that implement the methods described herein. Those engines may be stored as sets of non-transitory computer-executable instructions. The computing device may be a distributed computing system, such as an Amazon Web Services, Google Cloud Platform Microsoft Azure, or other public, private and/or hybrid cloud computing solution.

The computing device includes a network interface communicatively coupled to network, for communicating to and/or from a portable personal computer, smart phone, electronic document, tablet, and/or desktop personal computer, or other computing devices. The computing device further includes an I/O interface connected to devices, such as digital displays, user input devices, etc.

The functions of the engines may be implemented across distributed computing devices, etc. connected to one another through a communication link. In other examples, functionality of the system may be distributed across any number of devices, including the portable personal computer, smart phone, electronic document, tablet, and desktop personal computer devices shown. The computing device may be communicatively coupled to the network and another network. The networks may be public networks such as the Internet, a private network such as that of a research institution or a corporation, or any combination thereof. Networks can include, local area network (LAN), wide area network (WAN), cellular, satellite, or other network infrastructure, whether wireless or wired. The networks can utilize communications protocols, including packet-based and/or datagram-based protocols such as Internet protocol (IP), transmission control protocol (TCP), user datagram protocol (UDP), or other types of protocols. Moreover, the networks can include a number of devices that facilitate network communications and/or form a hardware basis for the networks, such as switches, routers, gateways, access points (such as a wireless access point as shown), firewalls, base stations, repeaters, backbone devices, etc.

The computer-readable media may include executable computer-readable code stored thereon for programming a computer (for example, comprising a processor(s) and GPU(s)) to the techniques herein. Examples of such computer-readable storage media include a hard disk, a CD-ROM, digital versatile disks (DVDs), an optical storage device, a magnetic storage device, a ROM (Read Only Memory), a PROM (Programmable Read Only Memory), an EPROM (Erasable Programmable Read Only Memory), an EEPROM (Electrically Erasable Programmable Read Only Memory) and a Flash memory. More generally, the processing units of the computing device may represent a CPU-type processing unit, a GPU-type processing unit, a field-programmable gate array (FPGA), another class of digital signal processor (DSP), or other hardware logic components that can be driven by a CPU.

Throughout this specification, plural instances may implement components, operations, or structures described as a single instance. Although individual operations of one or more methods are illustrated and described as separate operations, one or more of the individual operations may be performed concurrently, and nothing requires that the operations be performed in the order illustrated. Structures and functionality presented as separate components in example configurations may be implemented as a combined structure or component. Similarly, structures and functionality presented as a single component may be implemented as separate components or multiple components.

Additionally, certain aspects are described herein as including logic or a number of routines, subroutines, applications, or instructions. These may constitute either software (e.g., code embodied on a machine-readable medium or in a transmission signal) or hardware. In hardware, the routines, etc., are tangible units capable of performing certain operations and may be configured or arranged in a certain manner. In example aspects, one or more computer systems (e.g., a standalone, client or server computer system) or one or more hardware modules of a computer system (e.g., a processor or a group of processors) may be configured by software (e.g., an application or application portion) as a hardware module that operates to perform certain operations as described herein.

In various aspects, a hardware module may be implemented mechanically or electronically. For example, a hardware module may comprise dedicated circuitry or logic that is permanently configured (e.g., as a special-purpose processor, such as a microcontroller, field programmable gate array (FPGA) or an application-specific integrated circuit (ASIC)) to perform certain operations. A hardware module may also comprise programmable logic or circuitry (e.g., as encompassed within a general-purpose processor or other programmable processor) that is temporarily configured by software to perform certain operations. It will be appreciated that the decision to implement a hardware module mechanically, in dedicated and permanently configured circuitry, or in temporarily configured circuitry (e.g., configured by software) may be driven by cost and time considerations.

Accordingly, the term "hardware module" should be understood to encompass a tangible entity, be that an entity that is physically constructed, permanently configured (e.g., hardwired), or temporarily configured (e.g., programmed) to operate in a certain manner or to perform certain operations described herein. Considering aspects in which hardware modules are temporarily configured (e.g., programmed), each of the hardware modules need not be configured or instantiated at any one instance in time. For example, where the hardware modules comprise a general-purpose processor configured using software, the general-purpose processor may be configured as respective different hardware modules at different times. Software may accordingly configure a processor, for example, to constitute a particular hardware module at one instance of time and to constitute a different hardware module at a different instance of time.

Hardware modules can provide information to, and receive information from, other hardware modules. Accordingly, the described hardware modules may be regarded as being communicatively coupled. Where multiple of such hardware modules exist contemporaneously, communications may be achieved through signal transmission (e.g., over appropriate circuits and buses) that connects the hardware modules. In aspects in which multiple hardware modules are configured or instantiated at different times, communications between such hardware modules may be achieved, for example, through the storage and retrieval of information in memory structures to which the multiple hardware modules have access. For example, one hardware module may perform an operation and store the output of that operation in a memory device to which it is communicatively coupled. A further hardware module may then, at a later time, access the memory device to retrieve and process the stored output. Hardware modules may also initiate communications with input or output devices, and can operate on a resource (e.g., a collection of information).

The various operations of the example methods described herein can be performed, at least partially, by one or more processors that are temporarily configured (e.g., by software) or permanently configured to perform the relevant operations. Whether temporarily or permanently configured, such processors may constitute processor-implemented modules that operate to perform one or more operations or functions. The modules referred to herein may, in some example aspects, comprise processor-implemented modules.

Similarly, the methods or routines described herein may be at least partially processor-implemented. For example, at least some of the operations of a method can be performed by one or more processors or processor-implemented hardware modules. The performance of certain of the operations may be distributed among the one or more processors, not only residing within a single machine, but also deployed across a number of machines. In some example aspects, the processor or processors may be located in a single location (e.g., within a home environment, an office environment or as a server farm), while in other aspects the processors may be distributed across a number of locations.

The performance of certain of the operations may be distributed among the one or more processors, not only residing within a single machine, but also deployed across a number of machines. In some example aspects, the one or more processors or processor-implemented modules may be located in a single geographic location (e.g., within a home environment, an office environment, or a server farm). In other example aspects, the one or more processors or processor-implemented modules may be distributed across a number of geographic locations.

Unless specifically stated otherwise, discussions herein using words such as "processing," "computing," "calculating," "determining," "presenting," "displaying," or the like may refer to actions or processes of a machine (e.g., a computer) that manipulates or transforms data represented as physical (e.g., electronic, magnetic, or optical) quantities within one or more memories (e.g., volatile memory, non-volatile memory, or a combination thereof), registers, or other machine components that receive, store, transmit, or display information.

As used herein any reference to "one aspect" or "an aspect" means that a particular element, feature, structure, or characteristic described in connection with the aspect is included in at least one aspect. The appearances of the phrase "in one aspect" in various places in the specification are not necessarily all referring to the same aspect.

Some aspects may be described using the expression "coupled" and "connected" along with their derivatives. For example, some aspects may be described using the term "coupled" to indicate that two or more elements are in direct physical or electrical contact. The term "coupled," however, may also mean that two or more elements are not in direct contact with each other, but yet still co-operate or interact with each other. The aspects are not limited in this context.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In addition, use of the "a" or "an" are employed to describe elements and components of the aspects herein. This is done merely for convenience and to give a general sense of the description. This description, and the claims that follow, should be read to include one or at least one and the singular also includes the plural unless it is obvious that it is meant otherwise.

This detailed description is to be construed as an example only and does not describe every possible aspect, as describing every possible aspect would be impractical, if not impossible. One could implement numerous alternate aspects, using either current technology or technology developed after the filing date of this application.

## Claims

1. A computer-implemented method for determining HER2-low status of a patient using molecular data of the patient, comprising:
receiving, via one or more processors, digital biological data;
processing, via one or more processors, the digital biological data corresponding to the patient using a trained multi-stage machine learning architecture, wherein the processing includes:
(i) processing the digital biological data using a trained HER2-positive model to determine whether the digital biological data indicates that a HER2 status of the patient is HER2-positive;
(ii) when the HER2 status of the patient is not HER2-positive, processing the digital biological data using a trained HER2-low model to identify whether the HER2 status of the patient is HER2-low; and
(iii) when the HER2 status of the patient is not HER2-positive or HER2-low, designating the HER2 status of the patient as HER2-negative;
generating, via one or more processors, a digital HER2-low status report corresponding to the patient; and
causing, via a display device, the digital HER2-low status report to be displayed.

2. The computer-implemented method of claim 1, wherein the digital biological data includes RNA data; and/or
wherein the digital biological data includes at least some transcriptomic data.

3. The computer-implemented method of claim 2, wherein the at least some of the transcriptomic data includes at least some data generated via RNA seq.

4. The computer-implemented method of any one of claims 1 to 3, wherein the digital biological data includes at least one of DNA data or copy number variant data; and/or
wherein receiving the digital biological data includes receiving the digital biological data from a next-generation sequencing platform.

5. The computer-implemented method of any one of claims 1 to 4, wherein the trained HER2-positive model is a random forest model; and/or
the trained HER2-low model is a random forest model; and/or
wherein the trained HER2-positive model is a binary classifier trained on molecular signature data labeled according to (HER2-positive, NOT-HER2-positive) labels; and/or
wherein the trained HER2-low model is a binary classifier trained on molecular signature data labeled according to (HER2-low, NOT-HER2-low) labels.

6. The computer-implemented method of any one of claims 1 to 5, further comprising:
generating a prediction as to the HER2-low, HER2-positive and/or HER2-negative status of a given sample based on the trained multi-stage machine learning architecture.

7. The computer-implemented method of any one of claims 1 to 6, further comprising:
identifying at least one patient from a population of patients by processing the data of the patient using trained multi-stage machine learning architecture; and
matching the identified patient for treatment using a targeted therapy.

8. The computer-implemented method of claim 7, wherein the targeted therapy is a HER2 targeted therapy.

9. The computer-implemented method of claim 8, wherein the targeted therapy is trastuzumab deruxtecan.

10. A computing system comprising:
one or more processors; and
one or more memories having stored thereon computer-readable instructions that, when executed, cause the computing system to:
receive digital biological data;
process the digital biological data corresponding to a patient using a trained multi-stage machine learning architecture, wherein the processing includes:
(i) processing the digital data using a trained HER2-positive model to determine whether the digital biological data indicates that a HER2 status of the patient is HER2-positive;
(ii) when the patient is not HER2-positive, processing the digital biological data using a trained HER2-low model to identify whether the HER2 status of the patient is HER2-low; and
(iii) when the patient is not HER2-positive or HER2-low, designating the HER2 status of the patient as HER2-negative;
generate, via one or more processors, a digital HER2-low status report corresponding to the patient; and
causing, via a display device, the digital HER2-low status report to be displayed.

11. The computing system of claim 10, wherein the digital biological data includes one or both of (i) RNA data, and (ii) at least some transcriptomic data.

12. The computing system of claim 11, wherein the at least some of the transcriptomic data includes at least some data generated via RNA seq.

13. A computer-readable medium having stored thereon computer-executable instructions that, when executed, cause a computer to:
receive digital biological data;
process the digital biological data corresponding to a patient using a trained multi-stage machine learning architecture, wherein the processing includes:
(i) processing the digital data using a trained HER2-positive model to determine whether the digital biological data indicates that a HER2 status of the patient is HER2-positive;
(ii) when the patient is not HER2-positive, processing the digital biological data using a trained HER2-low model to identify whether a HER2 status of the patient is HER2-low; and
(ii) when the patient is not HER2-positive or HER2-low, designating the HER2 status of the patient as HER2-negative;
generate, via one or more processors, a digital HER2-low status report corresponding to the patient; and
cause, via a display device, the digital HER2-low status report to be displayed.

14. The computer-readable medium of claim 13, wherein the digital biological data includes one or both of (i) RNA data, and (ii) at least some transcriptomic data.

15. The computer-readable medium of claim 14, wherein the at least some of the transcriptomic data includes at least some data generated via RNA seq.
